(19) Europäisches Patentamt
European Patent Office
Office européen des brevets

(11) **EP 2 934 415 B1**

(12) **EUROPEAN PATENT SPECIFICATION**

(45) Date of publication and mention
of the grant of the patent:
**04.04.2018 Bulletin 2018/14**

(51) Int Cl.:
**A61F 13/537** *(2006.01)*    **A61F 13/53** *(2006.01)*

(21) Application number: **12890405.9**

(22) Date of filing: **20.12.2012**

(86) International application number:
**PCT/SE2012/051460**

(87) International publication number:
**WO 2014/098677 (26.06.2014 Gazette 2014/26)**

(54) **ABSORBENT ARTICLE WITH INCREASED COMFORTABILITY**

SAUGFÄHIGER ARTIKEL MIT ERHÖHTEM KOMFORT

ARTICLE ABSORBANT PRÉSENTANT UN CONFORT AMÉLIORÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.10.2015 Bulletin 2015/44**

(73) Proprietor: **SCA Hygiene Products AB
405 03 Göteborg (SE)**

(72) Inventors:
• **ABBAS, Shabira
S-431 69 Mölndal (SE)**
• **BAGGER-SJÖBÄCK, Anna
S-414 63 Göteborg (SE)**
• **BURVALL, Angelica
S-517 37 Bollebygd (SE)**

• **PALMQVIST, Lisa
S-431 49 Mölndal (SE)**

(74) Representative: **Zacco Sweden AB
P.O. Box 5581
114 85 Stockholm (SE)**

(56) References cited:
EP-A2- 1 269 953        WO-A1-03/053300
WO-A1-2006/093439    US-A1- 2002 156 443
US-A1- 2002 156 443    US-A1- 2003 069 555
US-A1- 2003 093 050    US-A1- 2003 114 810
US-A1- 2004 267 226    US-A1- 2005 131 371
US-A1- 2005 136 773    US-A1- 2008 091 158
US-B1- 6 423 043

EP 2 934 415 B1

## Description

TECHNICAL AREA

The invention relates to an absorbent product such as a sanitary napkin, panty liner or an incontinence protector with a plane extension comprising a longitudinal direction, a transverse direction and a thickness direction, wherein the product comprises an absorption body, which absorption body comprises in its thickness direction a liquid-receiving, open-cell foam layer and a liquid-absorbent fibrous layer, wherein the liquid-receiving open-cell foam layer and the liquid-absorbent layer comprise two opposite, longitudinal side edges extending in the longitudinal direction and two opposite transverse edges extending in the transverse direction.

BACKGROUND

For absorbent products such as sanitary napkins, panty liners and incontinence protectors that are provided to rest against the user's body when used, there are high requirements that they are discreet, soft and comfortable to wear and at the same time have a reliable security against leakage.

A common problem associated with absorbent products of this type is that the product deforms during use since the product is pressed together between the user's thighs. This can bring it about that folds occur in an uncontrolled manner in the product. The folds bring it about that channels can form on the product surface and that the liquid runs out past the product's side edges and creates leakage. Furthermore, the compression of the product entails that the surface accessible for receiving liquid is reduced, whereby the risk that liquid ends up next to the product increases. The problem that undesired folds form commonly occurs, for example, when using airlaid, cellulose-based absorbent layers. In order to reduce the problem that the product deforms during use, in particular in the crotch part of the product, it proved to be advantageous to increase the stiffness of the product. Special materials with great stiffness, so-called stiffening elements, have been introduced into the product. Stiffening elements have the goal of retaining the shape of the product during use and of controlling the deformation in such a manner as to prevent the development of leakage. The stiffening elements can be substantially two-dimensional, three-dimensional or initially substantially two-dimensional but when the product is used they expand and form a three-dimensional structure. Documents that describe absorbent products comprising forming-and/or stiffening elements and able to retain their form under load are, for example, WO 98/22057, WO 98/22058, WO 98/22061 and WO 98/22062. US2003/0093050A1 describes an absorbent article comprising a foam fluid handling layer, EP1269953A2 describes an absorbent article comprising a foam absorbent layer, and US2002/156443A1 describes absorbent product comprising a stiffening element.

However, the skin in a user's crotch is relatively sensitive and a problem with using absorbent products with stiffening elements is that they can scrape the user's skin, for example, when the user moves. Furthermore, it is essential that the absorbent product is experienced to be reliable and that the user trusts that the product will function well as well as being soft and discreet to wear.

Furthermore, there is a risk that the user's skin becomes moist due to the fact that it becomes sealed around the user's genital area when using the absorbent product. It is a problem that the user experiences discomfort, and the risk of bacterial growth with accompanying skin and odour problems increases.

DESCRIPTION OF THE INVENTION

The problem with creating a product that is experienced as discreet, soft and comfortable to wear at the same time that it exhibits satisfactory security against leakage has essentially been removed by the present invention.

A product made in accordance with the invention is distinguished principally in that the liquid-receiving, open-cell foam layer has a total surface in the plane extension of the product that covers the entire surface of the liquid-absorbent, fibrous layer in the plane extension and that each of the longitudinally extending side edges of the liquid-receiving, open-cell foam layer extends at least along a part of its length outside of each of the longitudinally running side edges of the liquid-absorbent, fibrous layer, and that the liquid-receiving, open-cell foam layer has an air permeability that is greater than 200 $m^3/m^2/min$ and an opacity greater than 35%, and that the liquid-absorbent, fibrous layer has a bending stiffness greater than 2.0 newtons according to the modified Circular Bend Procedure method. An advantage of the fact that the liquid-absorbent layer has a bending stiffness greater than 2.0 newtons is that this creates a relatively stiff and stable product, so that that the liquid-absorbent layer also functions as a stiffening element. The bending stiffness brings it about that the product does not become so limp and that it cannot readily shrink together and create undesired folds in the crotch area. In order to simultaneously create a product that is soft, flexible and comfortable to wear, it proved to be essential that each longitudinal side edge of the liquid-receiving, open-cell foam layer extends at least along a part of its length outside of each longitudinally running side edge of the liquid-absorbent, fibrous layer, and that the liquid-receiving, open-cell foam layer has an air permeability that is greater than 200 $m^3/m^2/min$. The liquid-receiving foam layer is an open-cell, flexible and pliable structure and the foam's pliability and flexibility reduces the risk of scrapes on account of the stiff edge parts on the liquid-absorbent, fibrous layer. It turned out that liquid-receiving layers of airlaid, cellulose-based layers and liquid-receiving layers of non-woven material do not have the ability to reduce the negative effect of the stiff edges which a stiff cellulose-based absorption layer causes. Flexible foam materials can spring back, i.e., return to substantially their original shape after having been exposed to outer loading. Flexible foam materials also have a padding effect such that the foam material lines the stiff edges and creates a soft distancing element between the user's skin and the stiff edges of the liquid-absorbent layer.

Furthermore, the liquid-receiving foam layer is open-celled and in this manner retains a good liquid-receiving ability.

The liquid-receiving foam layer is an open-cell, continuous structure. Since the foam layer is a continuous structure, it exhibits good pliability and the ability to spring back following outer loading. On the contrary, fibre-and/or filament-based liquid-receiving layers consist of manifold discrete fibres and/or filaments that are often intermittently connected together to each other. However, the points where they are connected together do not create a continuous structure such as is created with a material formed by foam. Fibre- and/or filament-based layers therefore do not have as good an ability to spring back and resume their original form after an outer loading. Liquid-receiving open-cell and flexible foam layers have a good ability to resume their original form in all directions after an outer loading.

The pliability and the ability to spring back of the foam also brings it about that there is less risk for folds to be produced or that the layer shrinks in comparison to layers based on fibres and/or filaments.

The liquid-receiving, open-cell foam layer consists of polyolefin-based foam, polystyrene-based foam, PVC foam, polyvinyl alcohol foam, acrylate foam, for example, manufactured according to HIPE technology, polyurethane foam, epoxy foam, latex foam, urea-formaldehyde foam, melamine-formaldehyde foam, silicone foam, viscose foam, carboxymethyl cellulose (CMC) foam, starch form, chitosan foam, alginate foam, polyactide foam, polyglycolide foam and polycaprolactone foam.

The surface of the liquid-absorbent layer in the plane extension can have local differences in the bending stiffness. However, it is important that the bending stiffness over any area of the surface of the liquid-absorbent layer is greater than 2.0 newtons, i.e., the liquid-absorbent layer falls within claim 1 since the bending stiffness is greater than 2.0 newtons in accordance with the modified Circular Bend Procedure method in any area of the liquid-absorbent layer.

According to another embodiment the surface of the liquid-absorbent layer in the plane extension has at least two different areas with a bending stiffness that is greater than 2.0 newtons according to the modified Circular Bend Procedure method. It comprises the fact that the surface, that has a bending stiffness that is greater than 2.0 newtons according to the modified Circular Bend Procedure method, should be able to be measured on two different areas on the surface of the liquid-absorbent layer, which two areas can be located adjacent to one another or be placed separated from one another.

According to another embodiment the liquid-absorbent layer has a bending stiffness that is greater than 2.0 newtons according to the modified Circular Bend Procedure method over its entire surface.

It has proved to be an advantage especially for sanitary napkins that are to absorb menstrual liquid, which has a strong colour, that the absorbed liquid that is absorbed into the product is not very visible. Due to the fact that the liquid-receiving, open-cell foam layer has an opacity that is greater than 35%, it turned out that the user experiences the product as more pleasing than a product that has liquid-receiving layers that are not as opaque, for example, fibre layers of airy, non-woven material. It is also important that the user finds before using the product that it is a reliable and aesthetically pleasing product. Due to the fact that the liquid-receiving layer has an opacity that is greater than 35%, the difference in size of the layers in the absorption body is not seen as distinctly, which gives a more reliable impression of the product even before use.

According to another embodiment the liquid-receiving, open-cell foam layer has an opacity greater than 50%.

According to an embodiment each longitudinal side edge of the liquid-receiving foam layer extends at least 5.0 millimetres outside of each longitudinally running side edge of the liquid-absorbent layer. The width of the surface of the liquid-receiving foam material in the longitudinal direction of the product extending outside of the surface of the underlying liquid-absorbent layer should be adapted in such a manner that a good flexibility is obtained. It turned out that the width on each longitudinally running side edge of the liquid-receiving foam layer extending outside of the underlying absorbent layer is preferably between 5-15 mm. As was previously described, the flexible foam material has a padding effect such that the foam material lines the stiff edges. In order to obtain a good padding effect it turned out that this distance, 5-15 mm, is optimal. As was also described previously, the ability of the foam material to spring back, i.e., to return to substantially its original form after having been exposed to outer loading is also an important property. The foam material has the ability to regain its original form in all directions, not only in the direction of the thickness of the material.

According to an embodiment, each longitudinally running side edge of the open-cell, liquid-receiving foam layer extends outside of each longitudinally running side edge of the liquid-absorbent, fibrous layer along its entire length. An advantage of such an embodiment is that such a product is experienced as softer, airier and also as more aesthetically pleasing by some users.

According to an embodiment the total surface of the open-cell, liquid-receiving layer in its plane extension is at least 1.7 times as great as the total surface of the liquid-absorbent, fibrous layer in the plane extension of the product. It turned out that the total surface of the liquid-receiving layer in its plane extension should be at least 1.7 times as great as the surface of the liquid-absorbent layer in order to obtain a product with optimal airiness. For some products, for example for a panty liner, that does not need to be able to absorb a great volume of menstrual liquid, the total surface of the liquid-absorbent, fibrous layer does not need to be equally great, which brings it about that for such products it can be advantageous if the total surface of the liquid-receiving foam layer in its plane extension is at least 2.0 times as great as the total surface of the liquid-absorbent, fibrous layer in the plane extension of the product. By virtue of the fact that the open-cell, liquid-receiving foam layer has an opacity that is greater than 35%, the difference in size on the layers is not seen as clearly, which makes the product more aesthetically pleasing and gives the product a more reliable impression. Therefore, a high opacity of the liquid-receiving layer is especially advantageous for products with a large difference of the surface between the liquid-receiving layer and the liquid-absorbent layer.

A small surface of the liquid-absorbent layer can be desirable for certain products, in part for making them thin and discreet and in part for reducing the material cost. Furthermore, an advantage with a large difference on the surface between the liquid-receiving layer and the liquid-absorbent layer is that when the liquid-absorbent layer also functions as a shaping element that follows the body when it moves, a certain difference of the layers can be required for obtaining the desired shaping. According to another embodiment, the open-cell liquid-receiving foam layer has an air permeability that is at least 300 m$^3$/m$^2$/min. Such an air-permeable open-cell liquid-receiving foam layer reduces the risk that the product is experienced as tight and uncomfortable.

According to another embodiment, the liquid-absorbent fibrous layer has a bending stiffness that is greater than 3.0 newtons according to the modified Circular Bend Procedure method. The liquid-absorbent layer falls within this embodiment since the bending stiffness is greater than 3.0 newtons according to the modified Circular Bend Procedure method in any area of the liquid-absorbent fibrous layer, i.e. in at least one area of the liquid-absorbent fibrous layer.

According to yet another embodiment, the liquid-absorbent fibrous layer has a bending stiffness that is greater than 4.0 newtons according to the modified Circular Bend Procedure method. The liquid-absorbent layer falls within this embodiment since the bending stiffness is greater than 4.0 newtons according to the modified Circular Bend Procedure method in any area of the liquid-absorbent fibrous layer, i.e. in at least one area of the liquid-absorbent fibrous layer.

According to another embodiment, the open-cell liquid-receiving foam layer has a bending stiffness that is less than 0.3 newtons according to the modified Circular Bend Procedure method. The liquid-receiving open-cell foam layer falls within this embodiment since the bending stiffness is less than 0.3 newtons according to the modified Circular Bend Procedure method in any area of the liquid-receiving open-cell foam layer, i.e. in at least one area of the liquid-receiving open-cell foam layer. According to another embodiment, the open-cell liquid-receiving foam layer has a bending stiffness that is less than 0.25 newtons according to the modified Circular Bend Procedure method. The liquid-receiving layer falls within this embodiment since the bending stiffness is less than 0.25 newtons according to the modified Circular Bend Procedure method in any area of the liquid-receiving layer, i.e. in at least one area of the liquid-receiving layer.

According to an embodiment the absorbent product comprises a liquid-permeable surface material and a backsheet that is liquid-tight, wherein the absorption body is arranged between the liquid-permeable surface material and the liquid-tight backsheet, and that the liquid-receiving foam layer is placed against the liquid-permeable surface material and the liquid-absorbent, fibrous layer is placed against the liquid-tight backsheet.

However, it is also possible that the absorbent product does not have a separate liquid-permeable surface material. In such an embodiment the liquid-receiving layer is placed closest to the user when the product is being used and thus the surface closest to the user consists of a flexible foam. An advantage of such an embodiment, is that the surface closest to the user is soft, smooth and pliable and that the number of layers used in the product is less, which simplifies the manufacturing process and possibly also reduces the material cost.

The liquid-absorbent, fibrous layer extends preferably in the longitudinal direction of the product over the crotch part and at least in part over the front part. In the transition between the crotch part and the front part, the liquid-absorbent, fibrous layer preferably has a width (M) which is less than 40 mm. Furthermore, the side edges of the liquid-absorbent, fibrous layer diverge in the front part of the product in the direction from the crotch part to at least a little over the front part. The width (M) is preferably between 25-40 mm, and more preferably between 30-35 mm. The narrowest point between the user's legs is where the muscle group passes that has its origin on the inside of the bottom of the pelvis and its attachment along the thigh. This muscle group consists of the muscles adductor brevis, adductor longus and adductor magnus. Measurements show that the distance in the user's crotch area between this adductor muscle group on the left and right side is surprisingly similar for all people and is around 30 mm. Since the liquid-absorbent layer of the product is constructed in a relatively stiff fibrous material, the width of the crotch part on the liquid-absorbent layer, at least in the area that is to pass between the muscle groups of each leg, should not exceed 40 mm, therefore. A width (M) that is less than 40 mm also reduces the risk of undesirable fold formation in the liquid-absorbent, fibrous layer.

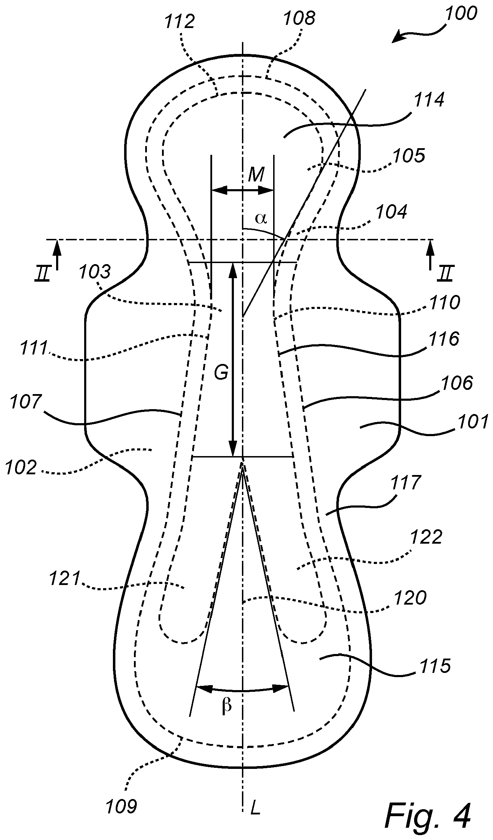

By designing the product with a sharply tapering part from the widest extension of the front part in the transverse direction of the product to the transition M between the front part and the crotch part, more transverse edges are obtained, see figure 4, which cling to the user's legs during use and prevent the product from gliding backwards. To obtain a good clinging effect, it proved suitable that the front part tapers from its full width to a width corresponding to the smallest available width in the user's crotch area in 20 mm of the product's length.

The absorbent product according to the invention is a sanitary napkin, a panty liner or an incontinence protector.

The product can be provided with a fastening element for fastening the product in a pair of pants. Suitable fastening elements are adhesive, for example, Velcro fastening surfaces, friction coatings or the like. The fastening element can also comprise side tabs or wings. Such side tabs or wings are folded around the crotch of the pants and attached to the pants or to one another.

## SHORT DESCRIPTION OF THE FIGURES

The invention will be described in detail in the following with reference made to the exemplary embodiments shown in the attached drawings. In the figures:

> Figure 1 shows a sanitary pad viewed from above;
> Figure 2 shows a section along line II-II through the sanitary napkin in figure 1.
> Figure 3 shows a section along line II-II through an alternative embodiment of the sanitary napkin in figure 1.
> Figure 4 shows another alternative embodiment of the sanitary napkin in figure 1 viewed from above.

## DESCRIPTION OF THE EMBODIMENTS

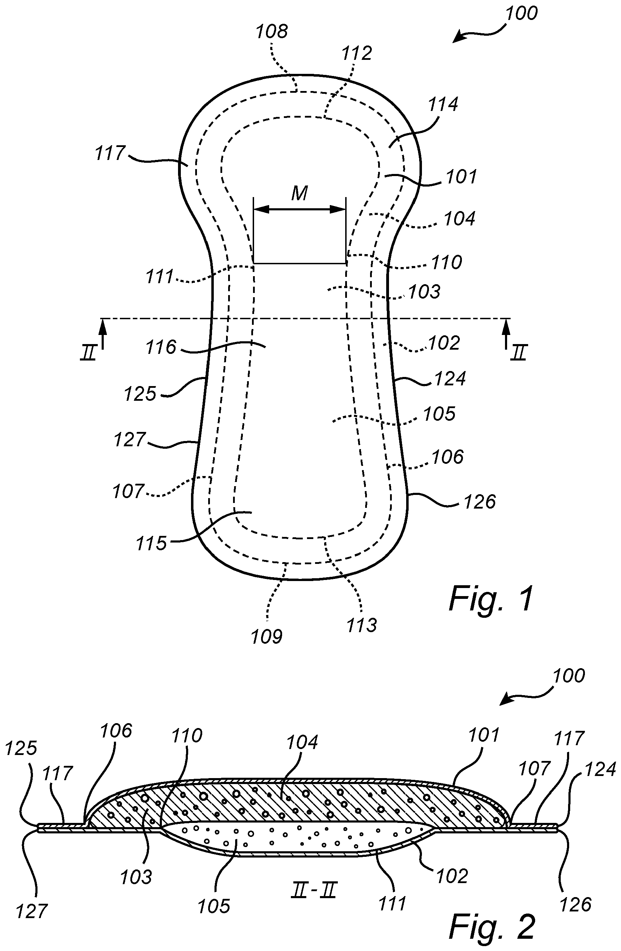

The sanitary napkin 100 shown in the figures 1 and 2 is elongated with a longitudinal direction and a transverse direction. The sanitary napkin 100 comprises a liquid-permeable surface material 101 arranged on the top side of the sanitary napkin, i.e., the side of the sanitary napkin intended to be turned toward a user during use, a liquid-tight backsheet 102, and an absorption body 103 arranged between the surface material 101 and the backsheet 102. The absorption body 103 comprises a liquid-receiving, open-cell foam layer 104 placed against the liquid-permeable surface material 101 and a liquid-absorbent fibrous layer 105 placed against the liquid-tight backsheet 102, whereby the liquid-receiving, open-cell foam layer 104 comprises two opposing, longitudinally running side edges 106, 107 extending in the longitudinal direction and two opposing transverse edges 108, 109 extending in the transverse direction, and the liquid-absorbent, fibrous layer 105 comprises two opposing longitudinally running side edges 110, 111 extending in the longitudinal direction, and two opposing transverse edges 112, 113 extending in the transverse direction. The liquid-receiving, open-cell foam layer 104 has a total surface in the plane extension of the product that covers the entire surface of the liquid-absorbent, fibrous layer 105 in its plane extension, and each longitudinally running side edge 106, 107 of the liquid-receiving foam layer 104 extends at least along a part of its length outside of each longitudinally running side edge 110, 111 of the liquid-absorbent, fibrous layer. Furthermore, the liquid-receiving open-cell foam layer 104 has a permeability to air that is greater than 200 $m^3/m^2/min$ and an opacity that is greater than 35%. The liquid-absorbent, fibrous layer 105 has a bending stiffness that is greater than 3.0 newtons.

The surface material 101 and the backsheet 102 have substantially the same plane form as the absorption body 103 but have a somewhat greater extension in the plane, as a result of which they form a projecting edge 117 around the entire periphery of the absorption body 103. Cover layers 102, 103 are mutually connected inside the projecting edge 117, for example, by gluing, sewing or welding with heat or ultrasound.

The sanitary napkin in figure 1 is formed with a front part 114, a back part 115 and an intermediate crotch part 116. The front part 114 has a rounded form and is wider than the crotch part 116. It turned out that all users have a critical area between the groins where the distance between the muscles running down on the inside of the thighs is approximately 30-35 mm. The width of the sanitary pad in the crotch area is limited in front by this distance between the muscle sinews right in front of the users' groins. Thus, in the transition between the crotch part 116 and the front part 114 the liquid-absorbent layer 105 has a distance M that does not exceed 40 mm and preferably is 30-35 mm. For the sanitary napkin 100 in accordance with our invention this is the width of the liquid-absorbent layer 105 in the transition between the crotch part 116 and the front part 114 that should not be too wide. The reason it is the width of the liquid-absorbent layer 105 in the crotch part 116 of the product which is most important is that it is this layer that contributes to the product's stiffness. In the direction back from this transition with width M to the end of the crotch part the width of the liquid-absorbent, fibrous layer 105, that also functions as a stiffening element, can continuously increase to a magnitude of size 1.5 times the width M.

A relatively narrow distance M, at least on the liquid-absorbent layer 105, is also an advantage from the viewpoint of fastening, since a sanitary pad where the difference in width between the front part 114 and the width of the narrowest part on the crotch part 116 is large, yields a good fastening effect against the user's legs and prevents the sanitary pad from gliding backwards during use.

The liquid-permeable surface material 101 suitably consists of a conventional liquid-permeable material. Examples of suitable materials are perforated plastic films, non-woven materials, plastic nets or the like.

The liquid-tight backsheet 102 is a conventional type and can thus consist of any liquid-tight material suitable for the purpose. Examples of such materials are various types of thin plastic films or non-woven materials treated to resist the penetration of liquid, for example, by being coated with plastic, wax or the like. Even other treatments such as heat calendering for melting a material that was permeable in the beginning to a mainly liquid-tight layer can be used. Furthermore, the liquid-tight backsheet 103 can consist of a liquid-tight surface on absorption body 103. In order to produce an airy product, it is common to use liquid-tight backsheet material that is breathable, i.e., has a good air permeability. According to the invention the liquid-tight backsheet material preferably consists of a breathable material. Examples of breathable materials are perforated films, microporous films, macroporous films, nanoporous films, monolithic films, fibrous non-woven ones and their laminates.

An example of a liquid-absorbent, fibrous layer 105 with a high absorption capacity and a good capacity for transporting liquid is the fibrous material described in WO 94/10953 and WO 94/10956. These materials are present in the form of dry-formed fibre layers with a high density and stiffness and are used directly in an absorbent product without being defibrated at first. The stiffening and absorbent element can also be made from a laminate of several non-woven layers or tissue layers that are mutually fixed for increased stiffness and that have highly absorbent particles between individual layers. The fixing of the individual layers to each other can take place with binding agents such as adhesive or melted fibres. The highly absorbent particles can also contribute to the bonding. The stiffness is controlled by selecting the number of layers and the amount of binding agent used and by the selection of highly absorbent material and how its adhesive capacity is utilized.

Another example of material in the liquid-absorbent layer is one or more layers of airlaid cellulose layers. This can be prefabricated material which is supplied in roll form for being then cut out in a suitable size, or alternatively the liquid-absorbent layer can be mat-formed and formed in-line during the manufacture itself of the absorbent product.

For example, to obtain compression lines/compression zones, there are areas that are compressed at a higher bending stiffness than the surrounding areas. Such a further compression can take place in combination with the compression of the liquid-absorbent layer. Alternatively, a pattern compression can take place in a separate step after the flat compression. The compression of the liquid-absorbent, fibrous layer can be made in several ways. An example of a common process is one that is called "high-density compression" (HDC), that is described in detail in EP-B-1427658. The compression can be made in two to three steps that include a preliminary compression and thereafter a compression in one or two steps. It is also possible to perform the compression in one step.

The surface of the liquid-absorbent layer in plane extension can exhibit local differences in bending stiffness. However, it is essential that the bending stiffness over one area on the surface of the liquid-absorbent layer is greater than 2.0 newtons, i.e., the liquid-absorbent layer falls within claim 1 if the bending stiffness is greater than 2.0 newtons according to the modified Circular Bend Procedure method in any area of the liquid-absorbent layer.

According to another embodiment the surface of the liquid-absorbent layer in its plane extension comprises at least two different areas with a bending stiffness that is greater than 2.0 newtons according to the modified bending stiffness method. It comprises the fact that the surface that has a bending stiffness greater than 2.0 newtons according to the modified Circular Bend Procedure method should be able to be measured on two different areas on the surface of the liquid-absorbent layer, which two areas can be adjacent to one another or can be placed at a distance from one another.

The liquid-absorbent layer preferably has a bending stiffness greater than 2.0 newtons according to the modified Circular Bend Procedure method over the entire liquid-absorbent layer. According to this embodiment no area on the liquid-absorbent layer may have a bending stiffness that is 2.0 newtons or lower when measured according to the modified Circular Bend Procedure method.

A suitable liquid-receiving, open-cell foam layer 104 is polyurethane foam. Polyurethane is a two-component product consisting of polyol and isocyanate that are mixed to polyurethane foam. Polyurethane has either open or closed cells. For being used as a liquid-receiving foam layer in an absorbent product, polyurethane foam is used with open cells. Polyurethane foam can have different stiffness and for our purpose flexible foams are used. The liquid-receiving foam is flexible with a low bending stiffness and springs back well, i.e., after being loaded the foam returns to substantially its original form.

The liquid-receiving, open-cell foam layer consists of polyolefin-based foam, polystyrene-based foam, PVC foam, polyvinyl alcohol foam, acrylate foam, for example, manufactured according to HIPE technology, polyurethane foam, epoxy foam, latex foam, urea-formaldehyde foam, melamine-formaldehyde foam, silicone foam, viscose foam, carboxymethyl cellulose (CMC) foam, starch foam, chitosan foam, alginate foam, polyactide foam, polyglycolide foam and polycaprolactone foam.

A fastening element in the form of a rectangular area extending in the longitudinal direction and consisting of self-adhesive glue is arranged on the outside of the liquid-tight backsheet layer. When the sanitary napkin 100 is being used, it is placed inside the user's underpants and is fastened in the underpants with the aid of the fastening element. Before being used, the fastening element is protected in a conventional manner, for example, by being covered by a protective layer of paper or plastic treated with silicone, or is embossed so that it can be readily separated from the glue when the sanitary napkin is to be used. The glue can obviously be alternatively arranged in any pattern suitable for the purpose such as a plurality of longitudinally running strands, an entire covering, arranged in areas only at the front part and/or the back part or the like. Furthermore, other types of fastening elements can be used such as friction covering, press studs, clamps, fastening flaps or the like.

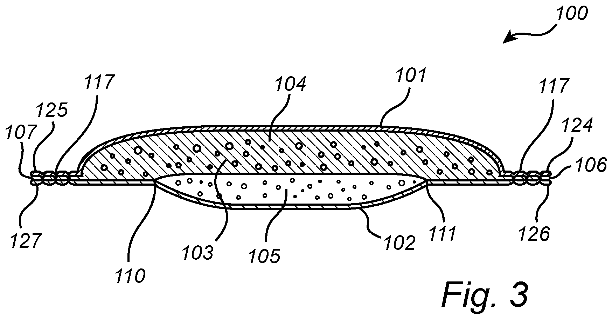

Figure 3 shows a transverse section of an alternative embodiment of the sanitary napkin 100 in accordance with the invention. The liquid-receiving foam layer 104 has a total surface in the plane extension of the product that is substantially the same as the surface of the liquid-permeable surface material 101 and substantially the same as the surface of the liquid-tight backsheet material 102. The liquid-permeable surface material 101 comprises two opposing, longitudinally running side edges 124, 125 extending in the longitudinal direction and the liquid-tight backsheet material 102 comprises two opposing longitudinally running side edges 126, 127 extending in the longitudinal direction. Thus, the two opposing longitudinal side edges 106, 107 of the open-cell, liquid-receiving foam layer 104, which side edges extend in the longitudinal direction, coincide substantially with the longitudinally running side edges 124, 125 of the liquid-permeable surface material and the longitudinally running side edges 126, 127 of the liquid-tight backsheet material. Thus, the surface material 101, backsheet material 102 and the liquid-receiving foam layer 104 comprise a projecting edge 117 around the entire periphery of the liquid-absorbent, fibrous layer 105. Cover layers 102, 103 and the liquid-receiving foam layer 104 are mutually connected inside the projecting edge 117, for example, by gluing, sewing or welding with heat or ultrasound. Therefore, the surface of the liquid-receiving foam layer 104 extends in its plane extension outside of each longitudinally running side edge 110, 111 of the liquid-absorbent, fibrous layer 105 and of each transversely running side edge 112, 113 of the liquid-absorbent, fibrous layer 105.

Furthermore, figure 4 shows an alternative embodiment of the sanitary napkin 100 in accordance with the invention. Figure 4 shows the sanitary napkin from the top. The liquid-absorbent, fibrous layer 105 comprises a shape like a keyhole. The liquid-receiving foam layer 104 also has a shape like a keyhole but with a greater extension in the longitudinal direction as well as in the transverse direction than the liquid-absorbent, fibrous layer 105. The liquid-absorbent, fibrous layer 105 is also intended to function as a stiffening element and is designed to reduce the risk that the sanitary napkin deforms in an uncontrolled manner. The liquid-absorbent, fibrous layer 105 has a size, shape and stiffness that bring it about that the product retains during the entire time of use a predetermined shape and furthermore is held fast at the intended location on the user.

The liquid-absorbent layer has a width M in the transition between the crotch part 116 and the front part 114 which width is less than 40 mm, preferably 30-35 mm. Both side edges of the front part 114 diverge in a forward direction in the product from this transition M. In this manner it is prevented that the product is shifted backwards between the user's legs. In figure 4 there is an angle between a line in the longitudinal direction of the product and each of the side edges of the liquid-absorbent, fibrous layer in the front part designated with $\alpha$. In the case of a large angle $\alpha$, for example, in the vicinity of 90°, the edges of the front part can scrape against the users groins and legs, thus creating discomfort for the user. The smaller the angle $\alpha$ is, the greater the risk that the product slides backwards in between the user's legs. At an angle below 30° this risk is unacceptably great. An angle between 35-45° gives the best balance between a firm hold and comfort.

The sanitary napkin 100 in figure 4 is formed with a crotch length adapted to the user's anatomy. In a sanitary napkin in accordance with the invention the fact was utilized that most women have a crotch length with a size of 80-100 mm. Therefore, the liquid-absorbent, fibrous layer 105 was created with a corresponding crotch length G with the size of 70-120 mm. Along the crotch, where the user's body shape is substantially plane, the sanitary napkin in accordance with the invention is shaped so as to be relatively stiff laterally, i.e., it is sufficiently stiff so as to not be deformed laterally and form folds. Since it is important that the liquid-absorbent, fibrous layer has a good absorption capacity, it is important to be able to utilize accessible space between the user's legs in the crotch. The width of the sanitary pad in the crotch area is limited in front by this distance between these muscle sinews right in front of the user's groins. In the direction backwards from these transition areas to the end of the crotch part the width of the liquid-absorbent, fibrous layer 105, that also functions as a stiffening element, can continuously increase to the size of 1.5 times the width M without the risk that the liquid-absorbent layer 105 scrapes the user in the crotch.

The liquid-absorbent layer 105 extends slightly over the product's back part 115. The liquid-absorbent layer 105 has a recess 120 in the back part 115 and extending from its end edge and in the direction toward the crotch part 116 by means of which the product can fold along a longitudinally running line in the recess and by means of which the parts, the legs 121 and 122, that are located on both sides of the recess 120, become more flexible than the wider crotch part 116. This recess 120 is for obtaining a good adaptation to and pliability with the body. The fold which develops in the recess when the product is being used can pass into the user's posterior notch, thus offering a very good protection against leakage via the posterior notch, which type of leakage occurs when using sanitary pads when the user is lying on her back. Furthermore, the recess 120 makes it possible that these legs 121, 122 can be mutually shifted vertically during various body movements, for example, when the user is walking.

In the exemplary embodiment shown in figure 4 the recess 120 is wedge-shaped and symmetrically located relative to the longitudinally running line of symmetry L of the product and forms an angle β with a magnitude of 20°. This angle can vary within broad limits but is of course dependent on the shape of the back part 115. The invention has been described above in connection with a sanitary napkin. It is possible, however, to utilise the invention also for panty liners and incontinence protectors.

EXAMPLES

The following materials were tested:

1. Polyurethane foam, FXI Foamex Innovations Inc., product code CAZ80A
2. Polyurethane foam, Foamex Innovations Inc., product code C80H2A
3. Polyurethane foam, FoamPartner, Reisgies Schaumstoffe GmbH, Regilen 30WF, item number 190321
4. Polyurethane foam, Caligen Foam Ltd., product code E 50
5. Melamine-based foam, BASF Plastics, Basotect W., product code E 2419 10
6. Polyurethane foam, Woodbridge Foam, product code SM25WH
7. Carded through, air-bound, non-woven, Fiberweb Tenotex, product code Airten 1250W6
8. Cellulose-based, multi-bound airlaid, Glatfelter Falkenhagen GmbH, product code MH080.137
9. Cellulose-based airlaid without SAP, "HDC" manufactured according to EP 1427658B1
10. Cellulose-based airlaid with 25 weight per cent SAP, "HDC" manufactured according to EP 1427658B1
11. Cellulose-based, multi-bound airlaid with 32 weight per cent SAP, Glatfelter Falkenhagen GmbH, product code VF250.103
12. Cellulose-based airlaid without SAP, manufactured according to WO 94/10953 with embossing pattern number 2072
13. Cellulose-based airlaid with 10 weight per cent SAP, manufactured according to WO 94/10953 with embossing pattern "Wave".

Explanations:

SAP = Super-absorbent particles manufactured from cross-bound and partially neutralized acrylic acid

Multi-bound airlaid consists primarily of cellulose fibres that are bound with tex bicomponent molten fibres and binding agent of ethylene vinylacetate copolymer "HDC" = High Density Compression is in the example a highly compressed, defibrated and mat-formed chemical sulphate mass with or without SAP

The materials 1-8 in examples 12 and 13 are based on fling-dried CTMP mass with or without SAP Materials 1-8 relate to liquid-receiving layers and materials 9-13 relate to liquid-absorbent layers. In the liquid-receiving layers 1-8 materials 1-6 relate to liquid-receiving, open-cell foam layers and materials 7 and 8 relate to liquid-receiving, fibrous layers. In the liquid-absorbent layers 9-13 all relate to liquid-absorbent, fibrous layers.

Density

The measurements were carried out in accordance with the EDANA method WSP 130.1.

In order to measure the average density of a material layer in an absorbent product it is essential that the various material layers included in the absorbent product are separated with caution. The material specimen to be measured is clipped out of the material layer. A loading pressure of 0.5 kPa is applied to the material specimen by a foot with an area of 45x45 mm and that is less than the area of the material specimen. Then the thickness of the material specimen is measured. The basis rate of the material sample, i.e., $gram/cm^2$, is weighed and calculated forward. Then, the density of the material specimen is calculated by dividing the base rate by the thickness.

Result:

| Material | Thickness | Density ($kg/m^3$) |
|---|---|---|
| 1 | 2.0 | 31 |
| 2 | 2.0 | 29 |
| 3 | 2.0 | 29 |
| 4 | 2.8 | 31 |
| 5 | 1.7 | 10 |
| 6 | 2.1 | 23 |
| 7 | 1.6 | 30 |
| 8 | 1.3 | 65 |
| 9 | 2.5 | 205 |
| 10 | 1.6 | 300 |
| 11 | 1.4 | 216 |
| 12 | 1.0 | 350 |
| 13 | 0.8 | 330 |

Air permeability

The measurements were carried out according to the EDANA method WSP 70.1. The apparatus used was the Textest FX 3300 from Testex Instruments. The apparatus measures what air permeability a material has in a defined pressure drop. We measured at a pressure drop of 200 Pa. The apparatus consists of a pump that sucks air through a hole and a measuring arm. The size of the hole is 20 $cm^2$. The air flow through the material is measured.

Result:

| Material | Air permeability |
|----------|------------------|
| 1 | 34 |
| 2 | 164 |
| 3 | 222 |
| 4 | 462 |
| 5 | 333 |
| 6 | 438 |
| 8 | 117 |

The value relates to the mean value from 10 measurements. The instrument's function was checked prior to the measurements against a standardised rubber plate that has a tightness of 104 l/m$^2$/sec at 200 Pa. The standardised rubber plate is available as an accessory from the instrument manufacturer.

Stiffness

Method description for modified Circular Bend Procedure:

The method for the modified Circular Bend Procedure was carried out according to a modified version of ASTM D 4032-82 (Circular Bend Procedure). The method for the modified Circular Bend Procedure is described in detail in EP 336 578. The measuring was carried out in the same manner as described in EP 336 578.
The apparatus that was used is the Instron Model No 5965. Instron Model No 5965 is manufactured by Instron Engineering Corporation. The bending resistance in the material sample was measured by measuring the maximum bending stiffness. According to the method, the maximum bending stiffness is constituted by a simultaneous deformation in several directions of a material sample, wherein one of the surfaces of the material sample becomes concave and the other surface of the material sample becomes convex. The method for the modified Circular Bend Procedure supplies a force value that concerns the bending resistance, i.e. simultaneous medium stiffness in all directions. The equipment used for the method of the modified Circular Bend Procedure is a modified Circular Bend Stiffness Tester that has the following parts:
A smooth-polished steel plate with measurements 102.0 x 102.0 x 6.35 mm that has a circular opening with a diameter of 18.75 mm. The nozzle has a bevelled edge that is 45 degrees to a depth of 4.75 mm. A pressure rod with a total length of 72.2 mm, a diameter of 6.25 mm and a spherical end with a radius of 2.97 mm was used. A sharp needle tip projects 0.88 mm out from the spherical end. The pressure rod was mounted concentrically and has the same magnitude of play in all directions. The end of the pressure rod is placed well over the plate with the circular opening. From this position the downwardly directed strike of the spherical tip is so long that it precisely extends to the bottom of the nozzle of the plate. However, an indication of the distance of the needle tip (0.88 mm) is not included. A tester of draught and compression with a loading cell adapted for Instron Model No. 5965 was used.

Production of test materials and calculation of the average value:

The liquid-receiving- respectively the liquid-absorbent material layer from five absorbent products was measured and then the average value was calculated.
In those cases where the liquid-absorbent layer comprises areas with different bending stiffness, for example, an area that is more compressed than an adjacent area, the liquid-absorbent layer falls within the scope of protection since any area has a bending stiffness that is greater than 2.0 newtons.
In those cases where the liquid-receiving foam layer comprises areas with different bending stiffness, for example, an area that is more compressed than an adjacent area, the liquid-receiving foam layer falls in a corresponding manner within the scope of protection since any area has a bending stiffness that is lower than that which is indicated in the claim.
When measuring material layers in an absorbent product, it is essential that the liquid-receiving foam layer is separated from the liquid-absorbent layer with great care so that the material layers do not break into pieces during the separating.

Implementation:

The implementation was made exactly in accordance with the description in EP 0 336 578 A1. The material specimens are stamped out or cut out and have an area of 37.5 x 37.5 mm. The material specimens were conditioned for two hours at a temperature of 21 ± 1°C and a relative air humidity of 50 ± 2%. The pressure rod should be moved downward at a rate of 50.0 cm/min.
The material specimen is then placed centred over the opening of the steel plate. The surface of the material specimen that is turned in the product towards the liquid-permeable surface material was turned in the taking of the specimen towards the pressure rod and the surface of the material sample that was turned in the product towards the liquid-impervious backside material was turned in the taking of the specimen towards the steel plate. The pressure plate was then put in motion and the maximum force was measured. The value of the maximum force was rounded off to the nearest gram.

Result:

| Material | Bending stiffness (newtons) |
|----------|------------------------------|
| 1 | 0.29 |
| 2 | 0.16 |

(continued)

| Material | Bending stiffness (newtons) |
|----------|-----------------------------|
| 3 | 0.20 |
| 4 | 0.10 |
| 5 | 0.27 |
| 6 | 0.18 |
| 7 | 0.07 |
| 8 | 0.59 |
| 9 | 3.43 |
| 10 | 2.28 |
| 11 | 3.40 |
| 12 | 6.55 |
| 13 | 2.94 |

Materials 1-8 are liquid-receiving layers. The result shows that layers 1-6 that are open-cell foam materials and also material 7, that is a carded through air-bound nonwoven material, have a bending stiffness that is lower than 0.30 newtons. In contrast thereto, material 8, which is a cellulose-based, multi-bound airlaid, exhibits a bending stiffness that is greater than 0.30 newtons.

Materials 9-13 are liquid-absorbent layers that exhibit a bending stiffness greater than 2.0 newtons.

The measurements were carried out in accordance with EDANA's method WSP 60.1.

Opacity is a concept and a magnitude in optics that is used to indicate transparency, that is, translucency. Opacity measures the degree of impenetrability for radiation through a material or transmission medium. Material that entirely lacks the ability to let light through is called opaque. The opacity was measured with a spectrophotometer with measurement in the YXY colour system (CIE 1931). The spectrophotometer used was of the brand Minolta Chroma Meter CR 300 (the y value was used) (CIE Illuminant D65). Calibration plate No. 16133079 was used as white standard background with a reflectance of 0.89. Black velvet was used as black standard background with a reflectance of 0.005.

The implementation was made in accordance with EDANA's method WSP 60.1.

The opacity (contrast ratio $C_0.89$) is calculated as follows:

$$\text{Contrast ratio } C_0.89 = R_b/R_w \times 100$$

$R_b$ = brightness, a specimen piece against black standard background
$R_w$ = brightness, a specimen piece against white standard background

The results are indicated with accuracy in whole numbers.

Result:

| Material | Opacity (contrast ratio $C_0.89$, %) |
|----------|--------------------------------------|
| 1 | 57 |
| 2 | 37 |
| 3 | 39 |
| 5 | 48 |
| 6 | 37 |
| 7 | 67 |
| 8 | 31 |

The result shows that the foam materials, i.e., materials 1, 2, 3, 5 and 6 have an opacity above 35%. Material 7, which is a cellulose-based material, also has an opacity that is over 35%. In contrast thereto, material 8, that is a carded through, air-bound non-woven material has a lower opacity, 31 %.

Foam materials 1, 2, 3, 5 and 6, as well as fibres/filaments in materials 7 and 8 are substantially white. However, it is also possible to have the materials in the layers coloured in other colours.

## Claims

1. An absorbent product (100) with a plane extension comprising a longitudinal direction, a transverse direction and a thickness direction, wherein the product comprises an absorption body (103), which absorption body (103) comprises in its thickness direction a liquid-receiving, open-cell foam layer (104) and a liquid-absorbent fibrous layer (105), wherein the liquid-receiving open-cell foam layer (104) and the liquid-absorbent fibrous layer (105) comprise two opposite, longitudinally running side edges (106, 107; 110, 111) extending in the longitudinal direction and two opposite transverse edges (108, 109; 112, 113) extending in the transverse direction, wherein the absorbent product is a sanitary napkin, a panty liner or an incontinence protector
**characterized in that** the liquid-receiving, open-cell foam layer (104) has a total surface in the plane extension of the product that covers the entire surface of the liquid-absorbent, fibrous layer (105) in the plane extension and that each of the longitudinally extending side edges (106, 107) of the liquid-receiving, open-cell foam layer (104) extends at least along a part of its length outside of each of the longitudinally running side edges (110, 111) of the liquid-absorbent, fibrous layer (105), and that the liquid-receiving, open-cell foam layer (104) consists of polyolefin-based foam, polystyrene-based foam, PVC foam, polyvinyl alcohol foam, acrylate foam, polyurethane foam, epoxy foam, latex foam, urea-formaldehyde foam, melamine-formaldehyde foam, silicone foam, viscose foam, carboxymethyl cellulose (CMC) foam, starch foam, chitosan foam, alginate foam, polyactide foam, polyglycolide foam or polycaprolactone foam, and has an air permeability that is greater than 200 m³/m²/min measured according to the EDANA method WSP 70.1 and an opacity greater than 35% measured according to the EDANA method WSP 60.1, and that the liquid-absorbent, fibrous layer (105) has a bending stiffness greater than 2.0 newtons, measured according to the modified Circular Bend Procedure method.

2. An absorbent product according to claim 1, wherein each longitudinally running side edge (106, 107) of the liquid-receiving, open-cell foam layer (104) extends at least 5.0 millimetres outside of each longitudinally running side edge (110, 111) of the liquid-absorbent, fibrous layer (105).

3. An absorbent product according to claim 1 or claim 2, wherein each longitudinally running side edge (106, 107) of the liquid-receiving, open-cell foam layer (104) extends outside of each longitudinally running side edge (110, 111) of the liquid-absorbent, fibrous layer (105) along its entire length.

4. An absorbent product according to any one of claims 1-3, wherein the total surface of the liquid-receiving, open-cell foam layer (104) in its plane extension is at least 1.7 times as great as the total surface of the liquid-absorbent, fibrous layer (105) in the plane extension of the product.

5. An absorbent product according to claim 4, wherein the total surface of the liquid-receiving, open-cell foam layer (104) in its plane extension is at least 2.0 times as great as the total surface of the liquid-absorbent, fibrous layer (105) in the plane extension of the product.

6. An absorbent product according to any one of the previous claims, wherein the liquid-receiving, open-cell foam layer (104) has an air permeability that is at least 300 m³/m²/min.

7. An absorbent product according to any one of the previous claims, wherein the liquid-absorbent, fibrous layer (105) has a bending stiffness that is greater than 3.0 newtons, more preferably greater than 4.0 newtons.

8. An absorbent product according to any one of the previous claims, wherein the liquid-receiving, open-cell foam layer (105) has a bending stiffness that is lower than 0.30 newtons.

9. An absorbent product according to any one of the previous claims, wherein the liquid-receiving, open-cell foam layer (105) has an opacity greater than 50%.

10. An absorbent product according to any one of the previous claims, wherein the product comprises a liquid-permeable surface material (101) and a backside material (102) that is liquid-tight, wherein the absorption body (103) is arranged between the liquid-permeable surface material (101) and the liquid-tight backsheet material (102), and that the liquid-receiving, open-cell foam layer (104) is placed against the liquid-permeable surface material (101) and the liquid-absorbent, fibrous layer (105) is placed against the liquid-tight backsheet material (102).

11. An absorbent product according to any one of the previous claims, wherein the product furthermore comprises a front part (114), a back part (115) and a crotch part (116) located between the back part and the front part, wherein the liquid-absorbent, fibrous layer (105) extends in the longitudinal direction of the product over the crotch part (116) and at least a portion over the front part (114) and that it has a width (M) in the transition between the crotch part (116) and the front part (114) which width is less than 40 mm, and that the side edges of the liquid-absorbent, fibrous layer (105) diverge in the direction from the transition between the crotch part (116) and the front part (114) to at least a portion over the front part (114).

## Patentansprüche

1. Saugfähiges Produkt (100) mit einer ebenen Ausdehnung, die eine Längsrichtung, eine Querrichtung und eine Dickenrichtung aufweist, wobei das Produkt einen saugfähigen Körper (103) aufweist, der saugfähige Körper (103) in seiner Dickenrichtung eine flüssigkeitsaufnehmende, offenzellige Schaumlage (104) und eine flüssigkeitsabsorbierende Faserlage (105) aufweist, die flüssigkeitsaufnehmende, offenzellige Schaumlage (104) und die flüssigkeitsabsorbierende Faserlage (105) zwei gegenüberliegende, in Längsrichtung verlaufende Seitenkanten (106, 107; 110, 111), die sich in der Längsrichtung erstrecken, und zwei gegenüberliegende Querkanten (108, 109; 112, 113) aufweist, die sich in der Querrichtung erstrecken, und das saugfähige Produkt eine Damenbinde, ein Pantyliner oder ein Inkontinenzschutz ist,
**dadurch gekennzeichnet, dass** die flüssigkeitsaufnehmende, offenzellige Schaumlage (104) eine Gesamtfläche in der ebenen Ausdehnung des Produkts aufweist, welche die gesamte Fläche der flüssigkeitsabsorbierenden Faserlage (105) in der ebenen Ausdehnung bedeckt, und das jede der sich in Längsrichtung erstreckenden Seitenkanten (106, 107) der flüssigkeitsaufnehmenden, offenzelligen Schaumlage (104) sich zumindest entlang eines Teils ihrer Länge außerhalb jeder der in Längsrichtung verlaufenden Seitenkanten (110, 111) der flüssigkeitsabsorbierenden Faserlage (105) erstreckt, und dass die flüssigkeitsaufnehmende, offenzellige Schaumlage (104) aus einem Schaum auf Polyolefinbasis, Schaum auf Polystyrolbasis, PVC-Schaum, Polyvinylalkoholschaum, Acrylatschaum, Polyurethanschaum, Epoxidschaum, Latexschaum, Harnstoff-Formaldehyd-Schaum, Melamin-Formaldehyd-Schaum, Silikonschaum, Viskose-Schaum, Carboxymethylcellulose-Schaum (CMC-Schaum), Stärkeschaum, Chitosanschaum, Alginatschaum, Polylactidschaum, Polyglycolidschaum oder Polycaprolactonschaum besteht und eine nach der EDANA-Methode WSP 70.1 gemessene Luftdurchlässigkeit aufweist, die größer als 200 m$^3$/m$^2$/min ist und eine nach der EDANA-Methode WSP 60.1 gemessene Opazität von größer als 35% aufweist, und dass die flüssigkeitsabsorbierende Faserlage (105) eine nach der modifizierten Circular-Bend-Procedure-Methode gemessene Biegesteifigkeit von größer als 2,0 N aufweist.

2. Saugfähiges Produkt nach Anspruch 1, bei dem sich jede in Längsrichtung verlaufende Seitenkante (106, 107) der flüssigkeitsaufnehmenden, offenzelligen Schaumlage (104) mindestens 5,0 mm außerhalb jeder in Längsrichtung verlaufenden Seitenkante (110, 111) der flüssigkeitsabsorbierenden Faserlage (105) erstreckt.

3. Saugfähiges Produkt nach Anspruch 1 oder 2, bei dem sich jede in Längsrichtung verlaufende Seitenkante (106, 107) der flüssigkeitsaufnehmenden, offenzelligen Schaumlage (104) außerhalb jeder in Längsrichtung verlaufenden Seitenkante (110, 111) der flüssigkeitsabsorbierenden Faserlage (105) entlang ihrer gesamten Länge erstreckt.

4. Saugfähiges Produkt nach einem der Ansprüche 1-3, bei dem die Gesamtfläche der flüssigkeitsaufnehmenden, offenzelligen Schaumlage (104) in ihrer ebenen Ausdehnung mindestens 1,7-mal größer ist als die Gesamtfläche der flüssigkeitsabsorbierenden Faserlage (105) in der ebenen Ausdehnung des Produkts.

5. Saugfähiges Produkt nach Anspruch 4, bei dem die Gesamtfläche der flüssigkeitsaufnehmenden, offenzelligen Schaumlage (104) in ihrer ebenen Ausdehnung mindestens 2,0-mal größer ist als die Gesamtfläche der flüssigkeitsabsorbierenden Faserlage (105) in der ebenen Ausdehnung des Produkts.

6. Saugfähiges Produkt nach einem der vorstehenden Ansprüche, bei dem die flüssigkeitsaufnehmende, offenzellige Schaumlage (104) eine Luftdurchlässigkeit aufweist, die zumindest 300 m$^3$/m$^2$/min beträgt.

7. Saugfähiges Produkt nach einem der vorstehenden Ansprüche, bei dem die flüssigkeitsabsorbierende Faserlage (105) eine Biegesteifigkeit aufweist, die größer als 3,0 N und vorzugsweise größer als 4,0 N ist.

8. Saugfähiges Produkt nach einem der vorstehenden Ansprüche, bei dem die flüssigkeitsaufnehmende, offenzellige Schaumlage (105) eine Biegesteifigkeit aufweist, die geringer als 0,3 N ist.

9. Saugfähiges Produkt nach einem der vorstehenden Ansprüche, bei dem die flüssigkeitsaufnehmende, offenzellige Schaumlage (105) eine Opazität von größer als 50% aufweist.

10. Saugfähiges Produkt nach einem der vorstehenden Ansprüche, wobei das Produkt ein flüssigkeitsdurchlässiges Flächenmaterial (101) und ein Rückseitenmaterial (102) aufweist, das flüssigkeitsundurchlässig ist, wobei der saugfähige Körper (103) zwischen dem flüssigkeitsdurchlässigen Flächenmaterial (101) und dem flüssigkeitsundurchlässigen Rückseitenmaterial (102) angeordnet ist und die flüssigkeitsaufnehmende, offenzellige Schaumlage (104) an dem flüssigkeitsdurchlässigen Flächenmaterial (101) platziert ist und die flüssigkeitsabsorbierende Faserlage (105) an dem flüssigkeitsundurchlässigen Rückseitenmaterial (102) platziert ist.

11. Saugfähiges Produkt nach einem der vorstehenden Ansprüche, wobei das Produkt darüber hinaus einen vorderen Teil (114), einen hinteren Teil (115) und einen zwischen dem hinteren Teil und dem vorderen Teil angeordneten Schrittteil (116) aufweist, sich die flüssigkeitsabsorbierende Faserlage (105) in der Längsrichtung des Produkts über den Schrittteil (116) und zumindest einen Abschnitt über den vorderen Teil (114) erstreckt und sie eine Breite (M) in dem Übergang zwischen dem Schrittteil (116) und dem vorderen Teil (114) aufweist, wobei die Breite weniger als 40 mm ist, und die Seitenkanten der flüssigkeitsabsorbierenden Faserlage (105) in der Richtung von dem Übergang zwischen dem Schrittteil (116) und dem vorderen Teil (114) mindestens in Richtung eines Abschnitts über dem vorderen Teil (114) auseinandergehen.

## Revendications

1. Produit absorbant (100) comportant une étendue plane comprenant une direction longitudinale, une direction transversale et une direction d'épaisseur, le produit comprenant un corps absorbant (103), lequel corps absorbant (103) comprend, dans sa direction d'épaisseur, une couche de mousse à alvéoles ouvertes recevant des liquides (104) et une couche fibreuse absorbant les liquides (105), la couche de mousse à alvéoles ouvertes recevant des liquides (104) et la couche fibreuse absorbant les liquides (105) comprenant deux bords latéraux opposés, s'étendant longitudinalement (106, 107 ; 110, 111) s'étendant dans la direction longitudinale et deux bords transversaux opposés (108, 109 ; 112, 113) s'étendant dans la direction transversale, le produit absorbant étant une serviette hygiénique, un protège-slip ou une protection d'incontinence, **caractérisé en ce que** la couche de mousse à alvéoles ouvertes recevant des liquides (104) a une surface totale dans l'étendue plane du produit qui couvre toute la surface de la couche fibreuse absorbant les liquides (105) dans l'étendue plane et **en ce que** chacun des bords latéraux s'étendant longitudinalement (106, 107) de la couche de mousse à alvéoles ouvertes recevant des liquides (104) s'étend, au moins sur une partie de sa longueur, à l'extérieur de chacun des bords latéraux s'étendant longitudinalement (110, 111) de la couche fibreuse absorbant les liquides (105) et **en ce que** la couche de mousse à alvéoles ouvertes recevant des liquides (104) se compose de mousse à base de polyoléfine, de mousse è base de polystyrène, de mousse de PVC, de mousse d'alcool polyvinylique, de mousse d'acrylate, de mousse de polyuréthane, de mousse d'époxyde, de mousse de latex, de mousse d'urée-formaldéhyde, de mousse de mélamine-formaldéhyde, de mousse de silicone, de mousse de viscose, de mousse de carboxyméthylcellulose (CMC), de mousse d'amidon, de mousse de chitosane, de mousse d'alginate, de mousse de polyactide, de mousse de polyglycolide ou de mousse de polycaprolactone, et a une perméabilité à l'air qui est supérieure à 200 $m^3/m^2/min$, mesurée selon la méthode EDANA WSP 70.1 et une opacité supérieure à 35 %, mesurée selon la méthode EDANA WSP 60.1, et **en ce que** la couche fibreuse absorbant les liquides (105) a une rigidité en flexion supérieure à 2,0 newtons, mesurée selon la méthode par essai de flexion circulaire (*Circular Bend Procedure*) modifiée.

2. Produit absorbant selon la revendication 1, dans lequel chaque bord latéral s'étendant longitudinalement (106, 107) de la couche de mousse à alvéoles ouvertes recevant des liquides (104) s'étend au moins 5,0 millimètres à l'extérieur de chaque bord latéral s'étendant longitudinalement (110, 111) de la couche fibreuse absorbant les liquides (105).

3. Produit absorbant selon la revendication 1 ou la revendication 2, dans lequel chaque bord latéral s'étendant longitudinalement (106, 107) de la couche de mousse à alvéoles ouvertes (104) recevant des liquides s'étend à l'extérieur de chaque bord latéral s'étendant longitudinalement (110, 111) de la couche fibreuse absorbant les liquides (105) sur toute sa longueur.

4. Produit absorbant selon l'une quelconque des revendications 1 à 3, dans lequel la surface totale de la couche de mousse à alvéoles ouvertes recevant des liquides (104) dans son étendue plane correspond à au moins 1,7 fois la surface totale de la couche fibreuse absorbant les liquides (105) dans l'étendue plane du produit.

5. Produit absorbant selon la revendication 4, dans lequel la surface totale de la couche de mousse à alvéoles ouvertes recevant des liquides (104) dans son étendue plane correspond à au moins 2,0 fois la surface totale de la couche fibreuse absorbant les liquides (105) dans l'étendue plane du produit.

6. Produit absorbant selon l'une quelconque des revendications précédentes, dans lequel la couche de mousse à alvéoles ouvertes recevant des liquides (104) a une perméabilité à l'air qui est d'au moins 300 $m^3/m^2/min$.

7. Produit absorbant selon l'une quelconque des revendications précédentes, dans lequel la couche fibreuse absorbant les liquides (105) a une rigidité en flexion qui est supérieure à 3,0 newtons, plus préférablement supérieure à 4,0 newtons.

8. Produit absorbant selon l'une quelconque des revendications précédentes, dans lequel la couche de mousse à alvéoles ouvertes recevant des liquides (105) a une rigidité en flexion qui est inférieure à 0,30 Newton.

9. Produit absorbant selon l'une quelconque des revendications précédentes, dans lequel la couche de mousse à alvéoles ouvertes recevant des liquides (105) a une opacité supérieure à 50 %.

10. Produit absorbant selon l'une quelconque des revendications précédentes, le produit comprenant un matériau de surface perméable aux liquides (101) et un matériau postérieur (102) qui est étanche aux liquides, le corps absorbant (103) étant disposé entre le matériau de surface perméable aux liquides (101) et le matériau postérieur étanche aux liquides (102), et la couche de mousse à alvéoles ouvertes recevant des liquides (104) étant disposée contre le matériau de surface perméable aux liquides (101) et la couche fibreuse absorbant les liquides (105) étant disposée contre le matériau postérieur étanche aux liquides (102).

11. Produit absorbant selon l'une quelconque des revendications précédentes, le produit comprenant en outre une partie avant (114), une partie arrière (115), et une partie correspondant à l'entrejambe (116) située entre la partie arrière et la partie avant, la couche fibreuse absorbant les liquides (105) s'étendant dans la direction longitudinale du produit par-dessus la partie correspondant à l'entrejambe (116) et sur au moins une partie de la partie avant (114), et ayant une largeur (M) dans la zone intermédiaire entre la partie correspondant à l'entrejambe (116) et la partie avant (114), laquelle largeur est inférieure à 40 mm, et les bords latéraux de la couche fibreuse absorbant les liquides (105) divergeant dans la direction de la zone intermédiaire entre la partie correspondant à l'entrejambe (116) et la partie avant (114) à au moins une partie de la partie avant (114).

Fig. 1

Fig. 2

Fig. 3

*Fig. 4*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9822057 A
- WO 9822058 A
- WO 9822061 A
- WO 9822062 A
- US 20030093050 A1
- EP 1269953 A2
- US 2002156443 A1

- WO 9410953 A
- WO 9410956 A
- EP 1427658 B
- EP 1427658 B1
- EP 336578 A
- EP 0336578 A1